# EUROPEAN PATENT APPLICATION

(11) **EP 0 663 159 A1**
(43) Date of publication of application: **19.07.1995**
(21) Application number: 93916251.7
(22) Date of filing: 02.08.1993
(51) Int. Cl.: A45D 33/00, A45D 34/00, A45D 40/00, A61L 2/02

(54) **COSMETICS STORAGE APPARATUS**

(71) Applicant: NIHONKENKOZOSHINKENKYUKAI CO. LTD., Fukuoka-shi Fukuoka 813 (JP)
(72) Inventor: MASUDA, Isamu Nihonkenkozoshinkenkyukai Co., Ltd., Fukuoka-shi Fukuoka 813 (JP)
(74) Representative: Dipl.-Phys.Dr. Manitz Dipl.-Ing. Finsterwald Dipl.-Ing. Grämkow Dipl.-Chem.Dr. Heyn Dipl.-Phys. Rotermund Morgan B.Sc.(Phys.)
(86) International application number: JP9301080
(87) International publication number: WO9503726

(57) **Abstract**

An apparatus for storing cosmetics such as a cleansing liquid, a toilet lotion, a milky lotion and a pack so that the quality thereof does not change. This apparatus is provided with storage spaces (7A, 7B, 7C) capable of holding cosmetics (6A, 6B, 6C) with their containers, and means for generating energy for promoting actions for reducing the contents of the cosmetics (6A, 6B, 6C) and minimizing the oxidation thereof, which energy generating means are arranged around the storage spaces (7A, 7B, 7C). When the energy works on the contents of the cosmetics (6A, 6B, 6C) held in the storage spaces (7A, 7B, 7C), the electrons constituting the substances of the contents are activated and fly out, and an electron-redundant condition, i.e. a reducing condition is generated, so that the oxidation of the contents of the cosmetics (6A, 6B, 6C) is minimized.

## Description

### Technical Field

This invention relates to a cosmetic keeping device which keeps cosmetics such as face cleansing lotion, face lotion, milky lotion, and face-pack so as to prevent them from deterioration.

### Background Art

Cosmetics such as face cleansing lotion, face lotion, milky lotion, and face-pack are generally filled into glass containers, synthetic resin containers, synthetic resin tubes, and so on. Since these cosmetics are used over a considerably long time, deterioration of the contents due to oxidation advances gradually. If the contents deteriorate due to oxidation, discolorations and generation of oxidation may result giving disagreeable feel to the user, and also may cause adverse effects to the users' skin by using.

### Disclosure of the Invention

A cosmetic keeping device according to the present invention has a housing space which can contain cosmetics with their containers, and around said housing space, an energy generating means which generates energy for accelerating reduction of contents of the cosmetics and inhibiting the contents from oxidation, is provided.

According to this invention, since reduction of the contents of cosmetics is accelerated and deterioration due to oxidation is inhibited by exerting energy from outside of the containers, discoloration of the contents and generation of oxide can be prevented, and also the quality of the cosmetics can be maintained over a long time. Therefore, there is no possibility of giving users disagreeable feel or having adverse effects on the users' skin.

### Brief Description of the Drawings

Fig. 1 is a perspective view showing an appearance of the cosmetic keeping device in one embodiment of the present invention.

Fig. 2 is a longitudinal section view of housing cases made of permanent magnets used in the embodiment of Fig. 1.

Fig. 3 is an explanatory view showing a state of magnetic fields exerting influence in the housing case.

Fig. 4 is an explanatory view showing a structure of an embodiment in which the housing cases made of permanent magnets are rotated.

Fig. 5 is a perspective view showing an appearance of a cosmetic keeping device in another embodiment of the invention.

### Best Mode for carrying out the Invention

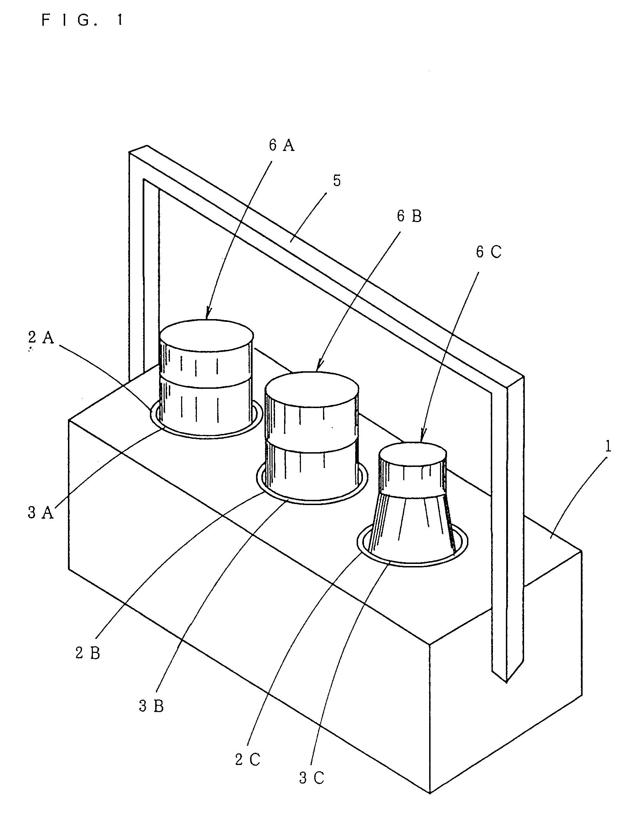

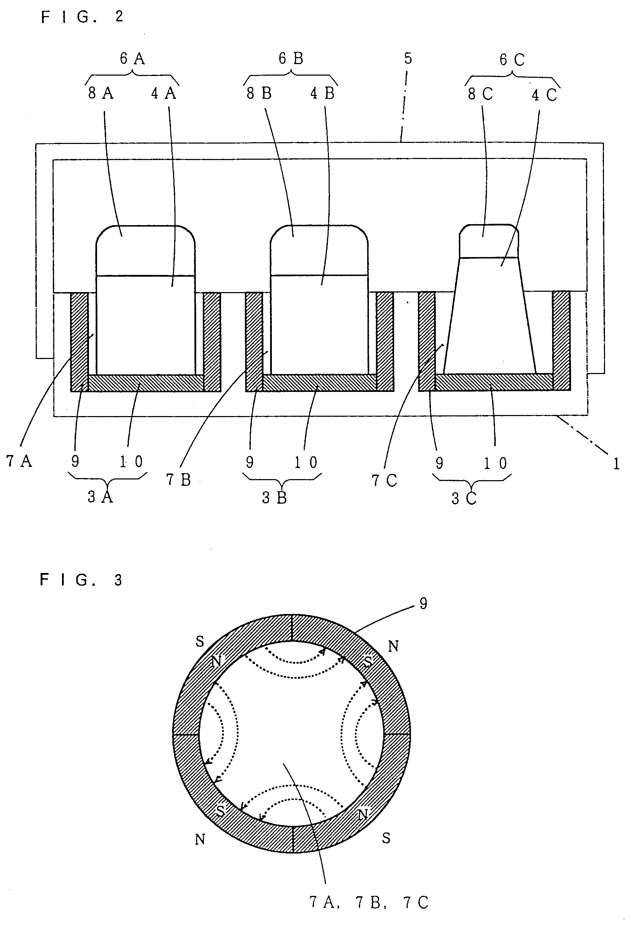

Fig. 1 shows an appearance of a cosmetic keeping device in one embodiment of the present invention. The example shown comprises a synthetic resin box type case 1 having a handle 5, and three holes 2A, 2B, and 2C on the top face of the case. In the holes 2A, 2B and 2C, cylindrical cases 3A, 3B, and 3C are inserted respectively, so that, as shown in Fig. 2, housing spaces 7A, 7B, and 7C which can contain cosmetics 6A, 6B, and 6C with their containers are formed in the cylindrical cases 3A, 3B, and 3C.

In this embodiment, the cosmetic 6A wherein face cleansing lotion are installed in a glass container 4A, the cosmetic 6B wherein face lotion are installed in a glass container 4B, and the cosmetic 6C wherein face-pack are installed in a synthetic resin tube 4C are respectively contained in the first cylindrical case 3A, in the second cylindrical case 3B and the third cylindrical case 3C. The containers 4A and 4B and the tube 4C are covered by caps 8A, 8B and 8C respectively. The cosmetics 6A, 6B and 6C are held in the cylindrical cases 3A, 3B and 3C respectively, with the caps 8A, 8B and 8C facing upward.

As shown in Fig. 2, each of the cylindrical cases 3A, 3B and 3C is a cylinder having an upper opening and a bottom. In this embodiment, the peripheral wall 9 of each cylindrical cases 3A, 3B and 3C is formed of a magnetic material, while each bottom 10 is formed of a non-magnetic material. As shown in Fig. 3, the peripheral wall 9 of each cylindrical cases 3A, 3B and 3C is so magnetized that an N-pole and an S-pole alternately appear on the inner surface, and D.C. magnetic fields (shown with dotted lined in the figure) are exerted toward the respective housing spaces 7A, 7B and 7C. The respective cylindrical cases 3A, 3B and 3C constitute energy generating means and accelerate reduction of the contents of the cosmetics 6A, 6B and 6C and inhibit oxidation, by said D.C. magnetic fields.

In addition, the method of magnetizing the peripheral wall 9 of each cylindrical cases 3A, 3B and 3C is, needless to say, not limited to the example shown. It is possible as required to change design, for example, the peripheral walls 9 are magnetized so that the N-pole appears on the inner surface, while the bottom 10 is formed of a permanent magnet wherein the S-pole appears on the inner surface. Furthermore, it is not necessarily required that the entire peripheral wall 9 of each of the cylindrical cases 3A, 3B and 3C is formed of permanent magnets, but a plurality of small pieces of permanent magnets may be suitably arranged.

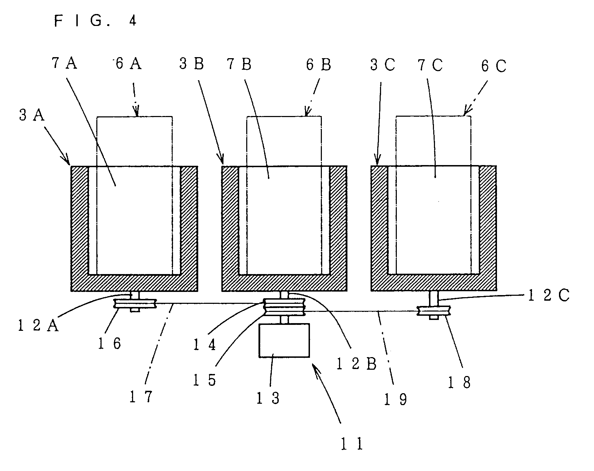

Fig. 4 is a configuration in which a rotation-drive device 11 is connected to the cylindrical cases 3A, 3B and 3C, and the respective cylindrical cases 3A, 3B and 3C pivotally rotate on respective rotation axes 12A, 12B and 12C. The rotation-drive device 11 has a low speed motor 13 as a drive-power source. This motor 13 is connected to said rotation axis 12B, on which two pulleys 14 and 15 are fixed. Between one pulley 14 and a pulley 16 fixed to the rotation axis 12A of the cylindrical case 3A, a belt 17 is wound, while between the other pulley 15 and a pulley 18 provided on the rotation axis 12C of the cylindrical case 3C a belt 19 is wound. When driving said motor 13, the three cylindrical cases 3A, 3B and 3C rotate simultaneously and generate revolving magnetic fields in the respective housing spaces 7A, 7B and 7C.

Besides rotating the D.C. magnetic field generator, in order to form a varying magnetic field in the respective housing spaces 7A, 7B and 7C, it Is also possible to use a plurality of solenoids instead of permanent magnets, and when flowing an A.C. current through each solenoid, an A.C. magnetic field can be exerted in the respective housing spaces 7A, 7B and 7C.

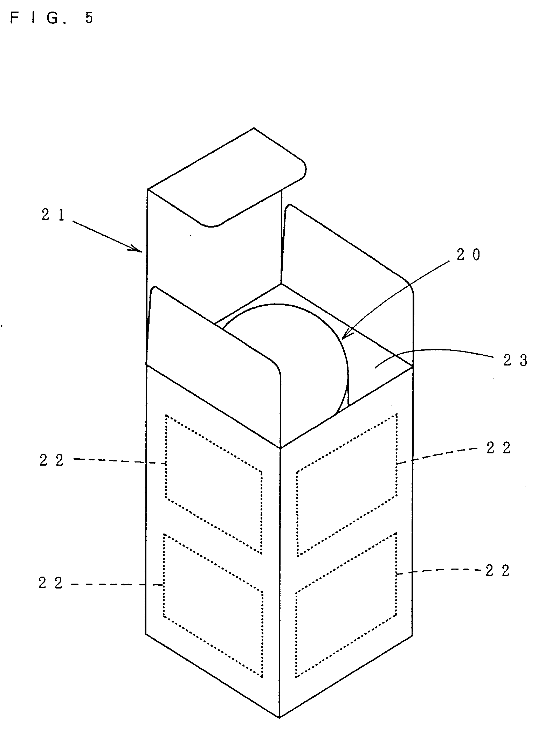

Fig.5 shows another embodiment of the invention. In the figure, a reference numeral 20 denotes a cosmetic, and 21 indicates a packing box containing the cosmetic 20. On the inner surface of this packing box 21, a plurality of rubber magnets 22 are attached with adhesive. In a housing space 23 inside said packing box 21, D.C. magnetic fields generated by the rubber magnets 22 are exerted, and the cosmetic 20 receives the effects of the D.C. magnetic fields, reduction is accelerated and oxidation is inhibited.

In addition, in the embodiments mentioned above, the magnetic field generating means which generate D.C. magnetic fields or A.C. magnetic fields are used as the energy generating means. The energy generating means are not limited to these, and far infrared ray or high frequency generating means may be used.

Moreover, in the embodiment of Fig.1, one part of each of three cosmetics 6A, 6B and 6C is projected from the corresponding housing space 7A, 7B or 7C. But it is of course possible to form each housing space that can enclose the entire contour of the corresponding cosmetic 6A, 6B or 6C.

### Industrial Applicability

In the embodiment of Fig. 1, when the cosmetic 6A, 6B and 6C are inserted into the respective cylindrical cases 3A, 3B and 3C of the box-type case 1, with the respective caps 8A, 8B and 8C facing upward, the respective cosmetics 6A, 6B and 6C are contained and kept in the respective cylindrical cases 3A, 3B and 3C. To the housing spaces 7A, 7B and 7C in respective cylindrical cases 3A, 3B and 3C, D.C. magnetic field generated by the permanent magnets are exerted, and when the magnetic energy acts on materials composing the cosmetics 6A, 6B and 6C, electrons around the nucleuses which constitute the materials are activated. As the result, rotational speed of electrons are increased, and the electron spins out and runs out. Thereby a state of surplus electrons, that is, a state of reduction is formed to lower the reduced potential, and oxidation of the contents of the cosmetics 6A, 6B and 6C is inhibited, keeping the qualities of them intact.

In the embodiment shown in Fig. 5, when the cosmetic 20 is contained in the packing box 21, the D.C. magnetic fields produced by the rubber magnets 22 exert to the cosmetic 20 to obtain the same effect as described above.

## Claims

1. A cosmetic keeping device comprising:
housing spaces capable of containing cosmetics with their containers, and an energy generating means provided around said housing spaces for generating energy for accelerating reduction of contents of the cosmetics and inhibiting the contents from oxidation.

2. A cosmetic keeping device in accordance with claim 1, wherein said energy generating means is a D.C. magnetic field generating means for having influence of D.C. magnetic fields on said housing spaces.

3. A cosmetic keeping device in accordance with claim 1 or 2, wherein said D.C. magnetic field generating means is comprised of permanent magnets which are fixedly arranged around the peripheries of said housing spaces.

4. A cosmetic keeping device in accordance with claim 1 or 2, wherein said D.C magnetic field generating means is comprised of permanent magnets which are movably arranged around the peripheries of said housing spaces.

5. A cosmetic keeping device in accordance with claim 1, wherein said energy generating means is an A.C. magnetic field generating means for having influence of A.C. magnetic fields on said housing spaces.

6. A cosmetic keeping device in accordance with claim 1, wherein said energy generating means is a far infrared ray generating means for having influence of far infrared rays on said housing spaces.
